(19)

**Europäisches Patentamt**

**European Patent Office**

**Office européen des brevets**

(11) **EP 3 876 825 B1**

(12) **EUROPEAN PATENT SPECIFICATION**

(45) Date of publication and mention
of the grant of the patent:
**03.12.2025 Bulletin 2025/49**

(21) Application number: **19813652.5**

(22) Date of filing: **07.11.2019**

(51) International Patent Classification (IPC):
*A61B 5/00* (2006.01)    *A61B 5/11* (2006.01)
*A61B 5/103* (2006.01)

(52) Cooperative Patent Classification (CPC):
**A61B 5/6892; A61B 5/1036; A61B 5/1116;**
**A61B 5/1121; A61B 5/7275; A61H 1/005;**
**G16H 50/30;** A61B 5/1127; A61H 2201/164;
A61H 2201/5007; A61H 2201/5043;
A61H 2201/5061; A61H 2201/5092;
A61H 2203/0406; A61H 2230/62

(86) International application number:
**PCT/IB2019/059577**

(87) International publication number:
**WO 2020/095244 (14.05.2020 Gazette 2020/20)**

(54) **ROBOTIZED PROPRIOCEPTIVE POSTURAL SYSTEM WITH INTEGRATED THREE-DIMENSIONAL CAMERA**

ROBOTISIERTES, PROPRIOZEPTIVES HALTUNGSSYSTEM MIT INTEGRIERTER DREIDIMENSIONALER KAMERA

SYSTÈME POSTURAL PROPRIOCEPTIF ROBOTISÉ À CAMÉRA TRIDIMENSIONNELLE INTÉGRÉE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **08.11.2018 IT 201800010151**

(43) Date of publication of application:
**15.09.2021 Bulletin 2021/37**

(73) Proprietor: **Tecnobody S.p.A.**
**24044 Dalmine (BG) (IT)**

(72) Inventors:
• **MARCANDELLI, Stefano**
**24040 Stezzano (BG) (IT)**
• **CARMINATI, Alessandro**
**24040 Stezzano (BG) (IT)**

(74) Representative: **Gatti, Enrico et al**
**Giambrocono & C. s.p.a.**
**Via Bartolomeo Bono, 15**
**24121 Bergamo (IT)**

(56) References cited:
EP-A1- 3 017 761      KR-B1- 100 894 895
TW-A- 201 225 916     US-B1- 8 847 989
US-B1- 9 149 222      US-B2- 9 868 026

• LIU T ET AL: "A wearable force plate system to successively measure multi-axial ground reaction force for gait analysis", ROBOTICS AND BIOMIMETICS (ROBIO), 2009 IEEE INTERNATIONAL CONFERENCE ON, IEEE, PISCATAWAY, NJ, USA, 19 December 2009 (2009-12-19), pages 836 - 841, XP031641859, ISBN: 978-1-4244-4774-9
• DUTT-MAZUMDER AVIROOP ET AL: "Transitions of postural coordination as a function of frequency of the moving support platform", HUMAN MOVEMENT SCIENCE, ELSEVIER, AMSTERDAM, NL, vol. 52, 16 January 2017 (2017-01-16), pages 24 - 35, XP029970039, ISSN: 0167-9457, DOI: 10.1016/J.HUMOV.2017.01.006

**(Cont. next page)**

- **JI-HYUN KO ET AL: "Transition of COM-COP relative phase in a dynamic balance task", HUMAN MOVEMENT SCIENCE, vol. 38, 1 December 2014 (2014-12-01), AMSTERDAM, NL, pages 1 - 14, XP055595701, ISSN: 0167-9457, DOI: 10.1016/j.humov.2014.08.005**

**Description**

The present invention refers to a robotized proprioceptive postural system with integrated three-dimensional camera, and in particular a system and a method for evaluating the coordination (balance) of a patient comprising a proprioceptive sensorized and robotized footboard.

Numerous studies have been carried out regarding the movement of the human body during the task of maintaining a static or dynamic equilibrium. In particular, as regards coordination of the body on a mobile platform or proprioceptive footboard, different models have been identified.

A first model consists in stiffening ankles, knees and hips so that the body functions as one single rigid segment, hence the name rigid mode pattern or ride pattern (Buchanan, J. J., & Horak, F. B. (1999), Emergence of postural patterns as a function of vision and translation frequency, Journal of Neurophysiology, 81(5), 2325-2339).

A second model is called ankle strategy (also called inverted pendulum model): using this pattern the patients stabilize their centre of mass by moving their body around their ankles. A further model is called hip strategy, during which the patients stabilize their centre of mass by alternately activating their thigh muscles and trunk muscles, thus using their hips to maintain balance (Horak, F. B., & Nashner, L. M. (1986), Central programming of postural movements: Adaptation to altered support-surface configurations, Journal of Neurophysiology, 55(6), 1369-1381).

In a last model, the patients jointly move hips, knees and ankles to maintain their erect position on an unstable platform (ankle-knee-hip strategy) (Ko, Y.-G., Challis, J. H., & Newell, K. M. (2001), Postural coordination patterns as a function of dynamics of the support surface, Human Movement Science, 20 (6), 737-764).

Studies concerning the equilibrium of the human body have always focused their attention on the possible parameters that could be used to better synthesise the behaviour of a patient in carrying out dynamic balance exercises.

Recent studies have identified as an identification variable the relative COP (Centre of Pressure) and COM (Centre of Mass) phase, in terms of both anteroposterior disorders (Ko, J.H., Challis, J. H., & Newell, K. M. (2014), Transition of COM-COP relative phase in a dynamic balance task, Human Movement Science, 38, 1-14) and mediolateral disorders (Aviroop Dutt-Mazumder, Karl Newell (2017), Transitions of postural coordination as a function of frequency of the moving support platform, Human Movement Science 52 (2017) 24-35).

By analysing the COM of a patient performing balance exercises, we are able to observe the point corresponding to the mean value of the distribution of the patient's mass, while the COP represents the position where the patient's support on the footboard is concentrated. For this reason, the COP - COM combination can be used to represent the global organization of a patient's postural system during the task he/she is carrying out.

History has seen a succession of increasingly innovative methods designed to evaluate a patient during specific equilibrium tests. A first method entailed for example the use of a force platform, on which a patient was subjected to perturbations by the therapist. From the force platform it was possible to evaluate the patient's COP behaviour but unfortunately the perturbations applied with this strategy were difficult to control since they consisted of pushing actions, making it very complicated to obtain repeatability between different tests and consequently compare the results obtained.

For evaluation of the COM, different techniques are used to try to evaluate the behaviour of the trunk and thus have a more detailed vision of the behaviour of the kinematic chain of the patient.

One way is to use an inertial sensor located on the trunk. With this method it is possible, for example, to estimate the flexions and extensions of the trunk on all three planes (frontal, sagittal and transversal). Unfortunately this method, in addition to being invasive for the user since it is applied on the body, usually by means of an elastic band, provides poor indications concerning the behaviour of the upper part of the body, and furthermore does not provide indications concerning the behaviour of the ankles, knees and hips.

To better monitor the movements of the upper part of the body, stereophotogrammetry is often used; the patient wears markers on given parts of the body and the robotic footboard is positioned inside a work area usually comprising 6 to 10 accurately calibrated cameras. The cameras recognise the markers positioned on the patient and by crossing the images detected at the same moment by two or more cameras, the spatial position of the marker can be identified; in this way it is possible to monitor the movements of the body and try to estimate the COM. Nevertheless, there are some problems connected with the use of this acquisition method: a first problem is connected with the markers that are applied on the patient which, although small, can be a nuisance for the person wearing them; however, limiting the number of positions in which they are applied also limits reconstruction of the patient's body during the specific exercises and makes calculation of the COM less accurate. A second problem concerns the need for considerable time to set up the system, the fact that the cameras require a purposely calibrated operating area and that the markers must be accurately positioned on the patient, making evaluation of the patient a fairly slow and laborious operation. A further problem is that the measurement depends on the operator, in fact the markers may be fixed in slightly different positions by different operators, thus modifying the results obtained. A further problem with the use of stereophotogrammetry is connected with the type of acquisition protocol used (e.g. Davis, Helen Hayes, etc.); each protocol entails different joint calculation methods, therefore different results will be obtained for the same patient.

US 8 847 989 B1 describes a force measurement system with a force measurement assembly configured to receive a subject thereon and at least one visual display device having an output screen. The force measurement assembly is rotatably mounted to a translatable sled assembly.

The object of the present invention is to provide a system comprising a proprioceptive footboard that allows the COM and COP to be determined and monitored.

A further object of the present invention is to provide a system that overcomes the drawbacks of the known art.

In accordance with the present invention, said objects and others are achieved by a system for evaluating the coordination of a patient according to claim 1.

Further characteristics of the invention are described in the dependent claims.

This solution offers various advantages compared to the solutions of the known art.

The system is formed of a proprioceptive footboard provided with force sensors able to measure the force applied by the patient on the footboard and two or more motors able to move the footboard with respect to the anteroposterior and mediolateral axes, and in any case rotate and translate the footboard until reaching 6 degrees of freedom.

It further comprises additionally or alternatively a robotic seat with force sensors able to measure the force applied by the patient on the seat and two or more motors also in this case which allow the seat to be rotated with respect to the two anteroposterior and mediolateral axes or the footboard to be rotated and translated.

Lastly, the system comprises a three-dimensional camera able to recognise the human body and estimate the positions of the body joints and in particular its centre of mass.

The system focuses on evaluation and training of the equilibrium of both normal-type patients and patients with an equilibrium deficit.

Using the robotized footboard it is possible to perturb as required the surface on which the patient rests and simultaneously film him/her with a three-dimensional camera able to recognize all the body segments and estimate the spatial positions of the joints, in particular the centre of mass, in order to carry out a complete evaluation of the patient's balancing capabilities. Furthermore, due to the three-dimensional training and improving their static and dynamic equilibrium capabilities.

The characteristics and advantages of the present invention will be evident from the following detailed disclosure of a practical embodiment thereof, illustrated by way of non-limiting example in the attached drawings, in which:

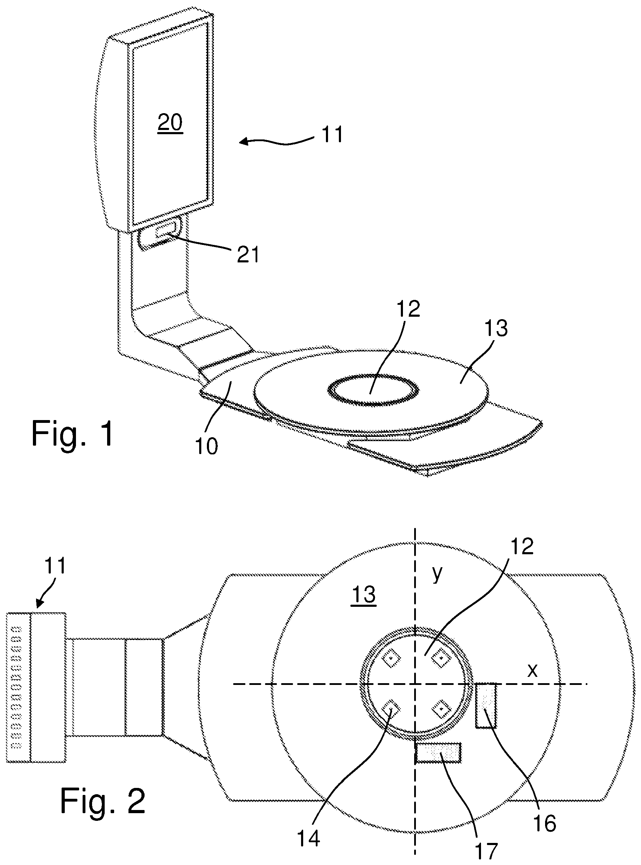

figure 1 shows a system comprising a proprioceptive footboard, seen in perspective, in accordance with a first embodiment of the present invention;
figure 2 shows a system comprising a proprioceptive footboard, seen from above, in accordance with a first embodiment of the present invention;

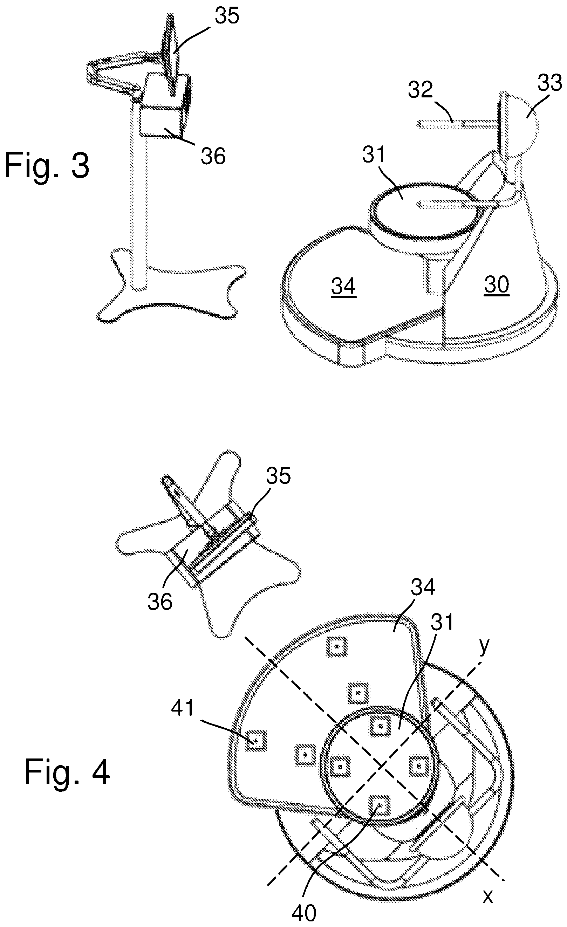

figure 3 shows a system comprising a proprioceptive footboard, seen in perspective, in accordance with a second embodiment of the present invention;
figure 4 shows a system comprising a proprioceptive footboard, seen from above, in accordance with a second embodiment of the present invention;
figures 5a and 5b show a graph of the trend of the COM and COP relative to two different perturbations of the patient.

Referring to the attached figures, a system for evaluating the coordination (equilibrium) of a patient, in accordance with a first embodiment that uses load cells as force sensors and that allows movement of the footboard only on the x and y axes of the present invention, comprises a substantially rectangular base 10. A frontal vertical structure 11 rises from one end of the base 10.

The base 10 houses a circular proprioceptive footboard 12, on which a user stands, surrounded by a safety platform 13, also circular.

The proprioceptive footboard 12 comprises four load cells 14 arranged in a cross formation and at 45° degrees with respect to the x and y axes of the base 10.

The load cells 14 allow determination of each single force applied to the proprioceptive footboard 12 and its intensity and the position of the centre of pressure (COP) are determined. The centre of pressure is evaluated considering the measurements taken by each single load cell 14.

In particular, the centre of pressure of the system formed of the four forces acting on the load cells 14 is calculated.

The distance from the centre of the proprioceptive footboard 12 to the centre 15 of the load cells 14 is called d and typically measures 0.175 m.

The load cells 14 are furthermore positioned at an angle of 45° with respect to the axes, and

$$\sigma = d * \cos(45)$$

The four load cells measure four force values F1, F2, F3, F4 which when added together correspond to the total force Ft exerted on the footboard.

At this point it is possible to calculate the factoring of the COP on the x and y axes, through the formulas:

$$Cx = \sigma * \frac{(F1 - F2 - F3 + F4)}{Ft}$$

$$Cy = \sigma * \frac{(F1 + F2 - F3 - F4)}{Ft}$$

It is also possible to calculate the arm of the moment applied by the patient on the footboard $r_{COP}$ and its angle $theta_{COP}$:

$$r_{COP} = sqrt(Cx^2 + Cy^2)$$

$$theta_{COP} = atan2(Cy, Cx)$$

In an alternative embodiment, sensors of different types can be used (for example piezoelectric, optical or optic fibre transducers can be used) and also in different numbers (three or more), with respect to those used in the embodiment described.

The proprioceptive footboard 12 is fixed to the base 10 by means of a hinge positioned in the centre 15 and can be rotated only along the x axis and the y axis by means of two motors 16 and 17.

In an alternative embodiment of the present invention, proprioceptive footboards can be used, produced and moved in a different way from those used in the embodiment described, for example different types of actuators can be used (brushless motors, direct current motors, pneumatic actuators, electro-mechanical actuators) and in variable numbers in order to obtain different movements (inclinations, rotations and translations).

On the vertical structure 11 at eye level is a system control and display panel 20 which is preferably a touch screen. It allows the display of information but can also receive input commands. Below the control panel 20 is a 3D detection system 21 for detecting the user's posture.

The 3D detection system 21 comprises a 3D camera and is able to recognise the human body and estimate the position of each joint at every moment by means of body tracking techniques suited to the needs of the present application. An example of body tracking technique is, for example, SDK Kinect which reconstructs the three-dimensional scene through an estimate of the depth map and recognises the human body within the area filmed, dividing it from the background; lastly, it estimates the spatial position of the body joints of the body detected.

The 3D camera consists of an rgb video camera and an infrared depth sensor, composed of an infrared laser scanner and a camera sensitive to the laser infrared. Via this sensor it is possible to obtain an rgb video image and a depth image. Some 3D cameras that can be used in this application are, for example: Kinect One (Microsoft, Redmond, USA), Astra Pro (Orbbec, Troy, USA), RealSense (Intel, Santa Clara, USA) and LIPSedge (LIPS, Taipei, Taiwan).

By means of the 3D display, the system 21 also identifies the person's movements which can be seen on the control panel 20.

A control centre (not shown) controls operation of the system, therefore receives the signals coming from the load cells 12 and from the 3D detection system 21, performs the necessary operations, drives the control panel 20 and the motors 16 and 17.

In an alternative embodiment of the present invention, the system comprises a base structure 30 which supports a circular proprioceptive footboard 31 positioned at a height such that the user can sit on it. It further comprises accessories that facilitate sitting such as armrests 32 and headrest 33.

At the front of the base structure 30 is a platform 34 on which the user can rest his/her feet.

Opposite the structure is a visor 35 and a 3D detection system 36 for detecting the posture of the user, arranged at the height of the user when seated.

Alternatively to the system composed of the elements 35 and 36, the control panel 20 of the system and the 3D detection system 21 for detecting the posture of the user, described previously, can be used.

The proprioceptive footboard 31 comprises four load cells 40 arranged in cross formation and at 45° degrees with respect to the x and y axes of the base structure 30.

The proprioceptive footboard 31 further comprises two motors (not shown) for the movement thereof in the directions of x and y, analogous to those defined by the numerical references 16 and 17.

Also the platform 34 comprises four load cells 41 arranged diverging along the x axis.

The system for evaluating the coordination (balance) of a patient using a motorised footboard, in accordance with the present invention, is used by standing the patient on the proprioceptive footboard 12 and operating the motors 16 and 17 which tilt the footboard, in such a way as to perturb the patient; said footboard can return to the initial position or can remain tilted, and the patient tries to keep his/her balance.

The 3D detection system 21 identifies the patient and sends the data to the control centre, like the data provided by the four load cells 14. The control centre calculates the COP and the COM, calculates the difference between them, and sends the results to the control and display panel 20.

In the case of the version with the proprioceptive footboard 31 where the patient sits, the procedure is as previously described. In addition, in this case it is also possible to verify the behaviour of the patient during the phase of standing up or sitting down on the proprioceptive footboard 31, by means of the sensors positioned on the proprioceptive footboard 31 and on the platform 34.

The use of a motorized footboard allows for controlled perturbations of the surface on which the patient evaluated is balancing; for example, it is possible to create perturbations on both the anteroposterior and mediolateral plane, or oscillations in a clockwise and anticlockwise direction, all with adjustable and perfectly repeatable amplitude and frequency.

The use of the three-dimensional camera allows recognition of the body and all its joints without having to apply any marker, therefore the measurements are no longer operator-dependent and patient evaluation times are significantly reduced.

Use of the system allows evaluation of the global behaviour of the body during balancing exercises; this is done, for example, by positioning the patient in a bipodalic position on the robotic footboard and by combining the data coming from the 3D camera and from the force sensors; the dynamics and kinematics of the body are evaluated (using COP, COM and positions of the joints) following perturbations of the footboard. Another example of use of the system allows peripheral evaluation, for example by positioning the patient on the robotized footboard and making him/her work in a monopodalic position with partial load, it is possible to evaluate his/her behaviour by exploiting the 3D camera and the force sensors of the footboard during exercises using one limb only. A further method of use is to position the patient on the seat and ask him/her to stand up, evaluating transfer of the load during the action of standing up.

The system can also perform a training function: by exploiting the camera, it is possible to provide patients with a biofeedback in real time giving them greater awareness of the spatial position of their body. The biofeedback is very useful for increasing the benefits of the training, enabling patients to improve their postural control, movement and force.

Furthermore, due to the combined use of the active footboard and the camera it is possible to immerse patients in a virtual reality subjecting them to multi-task exercises during which they are asked to carry out various tasks, for example pressing buttons while trying to keep their balance in bipodalic or monopodalic position, also following perturbations of the supporting surface. This mode is very useful for improving coordination and balance, consequently reducing the risk of falling.

The COM and COP data we have calculated in this way enable us to examine the postural control of a person using a more accurate, rapid and economic method than the known art. To examine the postural control, in fact, it is possible to evaluate the movement of the COM following a perturbation and in particular its position relative to the centre of pressure of the feet on the footboard (COP). In particular a very effective method for evaluating the postural control of a patient is to calculate the COP - COM difference at every moment. This difference is closely correlated with the horizontal acceleration of the body while it tries to keep its balance following a perturbation and represents what can be seen as the error which the person must compensate for in order to remain standing.

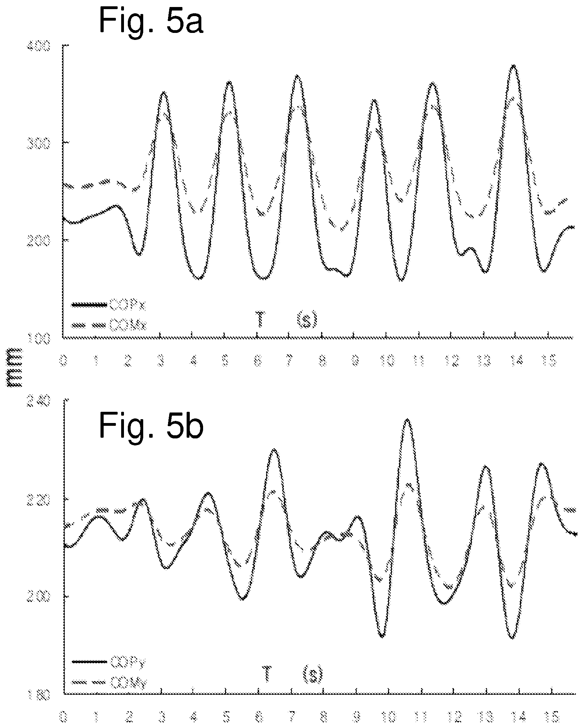

Figure 5 shows an example of behaviour of a patient, movement of the COP and COM in millimetres, with time variation in seconds, following two different perturbations.

The signal shown by a broken line is the COM and the signal shown by a continuous line is the COP.

The difference between the two signals, therefore COP - COM, represents the compensation which a patient has to make in order to achieve a stable position following the perturbation.

## Claims

1. A system for evaluating the coordination of a patient comprising: a mobile proprioceptive footboard (12, 31), on which said patient is located; wherein said proprioceptive footboard (12, 31) is fixed to a base (10) by means of a hinge positioned in the centre (15); wherein said system comprises two motors (16, 17) to rotate the proprioceptive footboard (12, 31) with respect to the anteroposterior axis and the mediolateral axis; said proprioceptive footboard (12, 31) comprises force sensors (14, 40, 41); a 3D detection system (21) configured to take a shot of said patient; wherein said 3D detection system (21) comprises a 3D camera consisting of an rgb camera and an infrared depth sensor; said 3D camera is able to recognise the human body of said patient and estimate the positions of the body joints and in particular its centre of mass; a control centre configured to receive the signals provided by said 3D detection system (21) and by said force sensors (14, 40, 41); said force sensors (14, 40, 41) comprise four load cells that are arranged in a cross formation and at 45° degrees with respect to the x and y axes of the base (10); said force sensors (14, 40, 41) allow determination of each single force applied to said proprioceptive footboard (12, 31) and its intensity and the position of the centre of pressure (COP) are determined; said control centre configured to calculate the centre of mass and the centre of pressure of said patient; said control centre configured to calculate the centre of pressure on the x and y axes, through the formulas:

$$Cx = \sigma * \frac{(F1 - F2 - F3 + F4)}{Ft}$$

$$Cy = \sigma * \frac{(F1 + F2 - F3 - F4)}{Ft}$$

wherein:

the distance from the centre of the proprioceptive footboard (12) to the centre (15) of the load cells (14) is called d;
with

$$\sigma = d * \cos(45);$$

the four load cells measure four force values F1, F2, F3, F4 which when added together correspond to the total force Ft exerted on the footboard;
a screen (20) configured to display the trends of said centre of mass and said centre of pressure of said patient.

2. The system according to claim 1 **characterised in that** said proprioceptive footboard (12, 31) is suitable for said patient to stand on.

3. The system according to one of the preceding claims **characterised in that** said proprioceptive footboard (12, 31) is suitable for said patient to sit on.

4. The system according to claim 3 **characterised in that** a platform (34) is placed in front of said proprioceptive footboard (12, 31), that is suitable for said patient to rest his/her feet, which comprises force sensors (14, 40, 41).

5. The system according to one of the preceding claims **characterised in that** said control centre is configured to calculate trend of said centre of mass and said centre of pressure of said patient and sends it to a display panel system.

6. The system according to one of the preceding claims **characterised in that** said control centre is configured to calculate the difference between said centre of mass and said centre of pressure of said patient.

7. The system according to claim 3 **characterised in that** the system comprises a base structure (30) which supports a circular proprioceptive footboard (31) positioned at a height such that the user can sit on it; the proprioceptive footboard (31) comprises four load cells (40) arranged in cross formation and at 45° degrees with respect to the x and y axes of the base structure (30).

**Patentansprüche**

1. System zum Bewerten der Koordination eines Patienten, umfassend: eine bewegliche propriozeptive Fußplatte (12, 31), auf der sich der Patient befindet; wobei die propriozeptive Fußplatte (12, 31) mittels eines Scharniers, das in der Mitte (15) positioniert ist, an einer Basis (10) befestigt ist; wobei das System zwei Motoren (16, 17) umfasst, um die propriozeptive Fußplatte (12, 31) in Bezug auf die anteroposteriore Achse und die mediolaterale Achse zu drehen; die propriozeptive Fußplatte (12, 31) Kraftsensoren (14, 40, 41) umfasst; ein 3D-Erfassungssystem (21) konfiguriert ist, um es eine Aufnahme des Patienten zu machen; wobei das 3D-Erkennungssystem (21) eine 3D-Kamera umfasst, die aus einer RGB-Kamera und einem Infrarot-Tiefensensor besteht; die 3D-Kamera in der Lage ist, den menschlichen Körper des Patienten zu erkennen und die Positionen der Körpergelenke und insbesondere seines Schwerpunkts zu schätzen; eine Steuerzentrale, die konfiguriert ist, um die Signale zu empfangen, die von dem 3D-Erfassungssystem (21) und von den Kraftsensoren (14, 40, 41) bereitgestellt werden; wobei die Kraftsensoren (14, 40, 41) vier Kraftmessdosen umfassen, die kreuzförmig und in einem Winkel von 45° zu der x- und y-Achse der Basis (10) angeordnet sind; die Kraftsensoren (14, 40, 41) eine Bestimmung jeder einzelnen Kraft ermöglichen, die auf die propriozeptive Fußplatte (12, 31) ausgeübt wird, und deren Stärke und die Position des Druckmittelpunkts (COP) werden bestimmt; wobei die Steuerzentrale konfiguriert ist, um den Massenschwerpunkt und den Druckschwerpunkt des Patienten zu berechnen; die Steuerzentrale konfiguriert ist, um den Druckmittelpunkt auf der x- und y-Achse anhand der folgenden Formeln zu berechnen:

$$Cx = \sigma * \frac{(F1 - F2 - F3 + F4)}{Ft}$$

$$Cy = \sigma * \frac{(F1 + F2 - F3 - F4)}{Ft}$$

wobei:
der Abstand von der Mitte der propriozeptiven Fußplatte (12) zu der Mitte (15) der Kraftmessdosen (14) als d bezeichnet wird;

$$\sigma = d * \cos(45);$$

mit

die vier Kraftmessdosen vier Kraftwerte F1, F2, F3, F4 messen, die miteinander addiert der Gesamtkraft Ft entsprechen, die auf die Fußplatte ausgeübt wird;
einen Bildschirm (20), der konfiguriert ist, um Tendenzen des Massenschwerpunkts und des Druckmittelpunkts des Patienten.

2. System nach Anspruch 1, **dadurch gekennzeichnet, dass** die propriozeptive Fußplatte (12, 31) geeignet ist, damit der Patient darauf steht.

3. System nach einem der vorherigen Ansprüche **dadurch gekennzeichnet, dass** die propriozeptive Fußplatte (12, 31) geeignet ist, damit der Patient darauf sitzt.

4. System nach Anspruch 3, **dadurch gekennzeichnet, dass** vor der propriozeptiven Fußplatte (12, 31) eine Plattform (34) platziert ist, die geeignet ist, damit der/die Patient/in seiner/ihrer Füße darauf stellen kann, die Kraftsensoren (14, 40, 41) umfasst.

5. System nach einem der vorherigen Ansprüche **dadurch gekennzeichnet, dass** die Steuerzentrale konfiguriert ist, um die Tendenz des Massenschwerpunkts und des Druckmittelpunkts des Patienten und diese an ein Anzeigetafelsystem zu senden.

6. System nach einem der vorherigen Ansprüche **dadurch gekennzeichnet, dass** die Steuerzentrale konfiguriert ist, um die Differenz zwischen dem Massenschwerpunkt und dem Druckmittelpunkt des Patienten.

7. System nach Anspruch 3, **dadurch gekennzeichnet, dass** das System eine Basisstruktur (30) umfasst, die eine kreisförmige propriozeptive Fußplatte (31) trägt, die in einer solchen Höhe positioniert ist, sodass der Benutzer darauf sitzen kann; die propriozeptive Fußplatte (31) vier Kraftmessdosen (40) umfasst, die kreuzförmig und in einem Winkel von 45° zu der x- und y-Achse der Basisstruktur (30) angeordnet sind.

**Revendications**

1. Système d'évaluation de la coordination d'un patient comprenant: un repose-pieds proprioceptif mobile (12, 31) sur lequel est placé ledit patient; dans lequel ledit repose-pieds proprioceptif (12, 31) est fixé à une base (10) au moyen d'une charnière positionnée au centre (15); dans lequel ledit système comprend deux moteurs (16, 17) permettant de faire tourner le repose-pieds proprioceptif (12, 31) par rapport à l'axe antéro-postérieur et à l'axe médio-latéral; ledit repose-pieds proprioceptif (12, 31) comprend des capteurs de force (14, 40, 41); un système de détection 3D (21) configuré pour prendre une image dudit patient; dans lequel ledit système de détection 3D (21) comprend une caméra 3D composée d'une caméra RGB et d'un capteur de profondeur infrarouge; ladite caméra 3D pouvant reconnaître le corps humain dudit patient et d'estimer les positions des articulations du corps et en particulier son centre de masse; un centre de contrôle configuré pour recevoir les signaux fournis par ledit système de détection 3D (21) et par lesdits capteurs de force (14, 40, 41); lesdits capteurs de force (14, 40, 41) comprennent quatre cellules de charge qui sont disposées en croix et à 45° par rapport aux axes x et y de la base (10); lesdits capteurs de force (14, 40, 41) permettent de déterminer chaque force individuelle appliquée audit repose-pieds proprioceptif (12, 31) ainsi que son intensité et la position du centre de pression (COP); ledit centre de contrôle est configuré pour calculer le centre de masse et le centre de pression dudit patient; ledit centre de contrôle est configuré pour calculer le centre de pression sur les axes x et y, à l'aide des formules suivantes:

$$Cx = \sigma * \frac{(F1 - F2 - F3 + F4)}{Ft}$$

$$Cy = \sigma * \frac{(F1 + F2 - F3 - F4)}{Ft}$$

dans lequel:

la distance entre le centre du repose-pied proprioceptif (12) et le centre (15) des cellules de charge (14) est appelée d;
avec

$$\sigma = d * \cos(45);$$

les quatre capteurs de force mesurent quatre valeurs de force F1, F2, F3, F4 qui, une fois additionnées, correspondent à la force totale Ft exercée sur le repose-pied; un écran (20) configuré pour afficher les tendances dudit centre de masse et dudit centre de pression dudit patient.

2. Système selon la revendication 1, **caractérisé en ce que** le repose-pieds proprioceptif (12, 31) est adapté pour que ledit patient puisse s'y tenir debout.

3. Système selon l'une des revendications précédentes **caractérisé en ce que** le repose-pieds proprioceptif (12, 31) est adapté pour que ledit patient puisse s'y assoir.

4. Système selon la revendication 3, **caractérisé en ce qu'**une plate-forme (34) est placée devant ledit repose-pieds proprioceptif (12, 31), et est adaptée pour que ledit patient puisse y poser ses pieds, et comprend des capteurs de force (14, 40, 41).

5. Système selon l'une des revendications précédentes, **caractérisé en ce que** ledit centre de contrôle est configuré pour calculer la tendance dudit centre de masse et dudit centre de pression dudit patient et l'envoie à un système de panneau d'affichage.

6. Système selon l'une des revendications précédentes, **caractérisé en ce que** ledit centre de contrôle est configuré pour calculer la différence entre ledit centre de masse et ledit centre de pression dudit patient.

7. Système selon la revendication 3, **caractérisé en ce**

**que** le système comprend une structure de base (30) qui prend en charge un repose-pieds proprioceptif circulaire (31) positionné à une hauteur de sorte que l'utilisateur puisse s'y asseoir; le repose-pieds proprioceptif (31) comprend quatre cellules de charge (40) disposées en croix et à 45° par rapport aux axes x et y de la structure de base (30).

Fig. 1

Fig. 2

Fig. 3

Fig. 4

## Fig. 5a

## Fig. 5b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8847989 B1

### Non-patent literature cited in the description

- **BUCHANAN, J. J.** ; **HORAK, F. B.** Emergence of postural patterns as a function of vision and translation frequency. *Journal of Neurophysiology*, 1999, vol. 81 (5), 2325-2339
- **HORAK, F. B.** ; **NASHNER, L. M.** Central programming of postural movements: Adaptation to altered support-surface configurations. *Journal of Neurophysiology*, 1986, vol. 55 (6), 1369-1381
- **CHALLIS, J. H.** ; **NEWELL, K. M.** Postural coordination patterns as a function of dynamics of the support surface. *Human Movement Science*, 2001, vol. 20 (6), 737-764
- **KO, J.H.** ; **CHALLIS, J. H.** ; **NEWELL, K. M.** Transition of COM-COP relative phase in a dynamic balance task. *Human Movement Science*, 2014, vol. 38, 1-14
- **AVIROOP DUTT-MAZUMDER** ; **KARL NEWELL**. Transitions of postural coordination as a function of frequency of the moving support platform. *Human Movement Science*, 2017, vol. 52, 24-35